# EUROPÄISCHE PATENTSCHRIFT

(11) **EP 0 763 047 B1**
(45) Veröffentlichungstag und Bekanntmachung des Hinweises auf die Patenterteilung: **23.01.2002**
(21) Anmeldenummer: 96912002.1
(22) Anmeldetag: 03.04.1996
(51) Int. Cl.: C07H 19/02

(54) **NEUES VERFAHREN ZUR HERSTELLUNG VON NUCLEOSIDEN**
NOVEL METHOD OF PRODUCING NUCLEOSIDES
NOUVEAU PROCEDE DE PRODUCTION DE NUCLEOSIDES

(30) Priorität: 03.04.1995 DE 19513330
(43) Veröffentlichungstag der Anmeldung: 19.03.1997
(73) Patentinhaber: HOECHST AKTIENGESELLSCHAFT, 65926 Frankfurt am Main (DE)
(72) Erfinder: VORBRÜGGEN, Helmut, D-13507 Berlin (DE); KROLIKIEWICZ, Konrad, D-12357 Berlin (DE); BENNUA-SKALMOWSKI, Bärbel, D-14167 Berlin (DE)
(86) Internationale Anmeldenummer: EP9601472
(87) Internationale Veröffentlichungsnummer: WO9631520

(56) Entgegenhaltungen:
- DATABASE WPI Week 7843 Derwent Publications Ltd., London, GB; AN 78-77591A XP002013049 & JP,A,53 108 986 (MITSUI TOATSU CHEM. INC.) , 22.September 1978 & JP,A,53 108 986
- TETRAHEDR. LETT., Bd. 36, Nr. 43, 23.Oktober 1995, Seiten 7845-8, XP002013048 H. VORBRÜGGEN ET AL.: "A New Simple Nucleoside Synthesis"

## Beschreibung

Die Erfindung betrifft ein neues Verfahren zur Herstellung von Nucleosiden.

Im deutschen Patent DBP 1919307 wird ein Verfahren zur Darstellung von Nucleosiden beschrieben, in dem O-acylierte 1-O-Acyl-, 1-O-Alkyl- sowie 1-Halozucker mit silylierten heterocyclischen Basen wie Uracil oder 6-Azauracil in Gegenwart von Friedel-Crafts Katalysatoren wie SnCl₄ zu den entsprechenden O-acylierten Nucleosiden umgesetzt werden.

Dieser Prozeß mit getrennter vorheriger Silylierung der heterocyclischen Basen sowie deren nachfolgende Kondensation mit den oben genannten O-acylierten 1-O-Alkyl- bzw. 1-O-Acylzuckern läßt sich nach DOS 25 08312 vereinfachen, indem die Silylierung der heterocyclischen Basen sowie ihre Kondensation mit O-acylierten 1-O-Alkyl- oder 1-O-Acylzuckern in einem Schritt durchgeführt werden.

JP-A-53108986 beschreibt die direkte Nukleosidsynthese aus persylierten Zuckern und persylierten Basen in Gegenwart von Zinntetrachlorid in Acetonitril.

Aber alle diese bisherigen Verfahren, die breite Anwendung in der Praxis gefunden haben, verwenden O-acylierte Zuckerderivate, die wie die sehr häufig verwendete 1-O-Acetyl-2,3,5-tri-O-benzoyl-ß-D-ribofuranose durch eine mehrstufige Synthese aus D-Ribose hergestellt werden muß.

Es wurde nun überraschenderweise gefunden, daß diese mehrstufige und aufwendige Umwandlung der freien Zucker wie z.B. der D-Ribose in die entsprechenden O-acylierten 1-O-Alkyl- oder 1-O-Acylzucker wie die bereits genannte 1-O-Acetyl-2,3,5-tri-O-benzoyl-ß-D-ribofuranose entfällt bzw. überflüssig wird, wenn man die freien Zucker wie D-Ribose oder D-Glucose in Gegenwart der freien Basen wie Uracil, 6-Azauracil, N⁶-Benzoyladenin oder N²-Acetylguanin in einem indifferenten Lösungsmittel mit Silylierungsmitteln wie Hexamethyldisilazan (HMDS) und Trimethylchlorsilan (TCS) persilyliert und gleichzeitig oder anschließend eine Lewissäure wie Trimethylsilyltriflat, Trifluormethansulfonsäure, Trimethylsilylnonaflat, Perfluorbutansulfonsäure oder SnCl₄ zusetzt, wobei die entsprechenden persilylierten Nukleoside entstehen. Diese persilylierten Nukleoside lassen sich durch Transsilylierung mit kochendem Methanol in die gewünschten freien Nukleoside überführen.

Gegenstand der Erfindung ist also ein neues Verfahren zur Nukleosidsynthese, dadurch gekennzeichnet, daß man den freien Zucker mit heterocyclischen Basen in Gegenwart von Silylierungsmitteln in einem inerten Lösungsmittel mit einer Lewissäure umsetzt.

Die erhaltenen persilylierten Nucleoside werden nach Neutralisation der Lewissäure durch Transsilylierung mit überschüssigem Methanol in die entsprechenden freien Nucleoside überführt.

Bei der Persilylierung der freien Zucker und der heterocyclischen Basen mit equivalenten Mengen an Hexamethyldisilazan (HMDS) und Trimethylchlorsilan (TCS) in siedendem absolutem Azetonitril sublimiert das sich bildende Ammoniumchlorid in den Rückflußkühler und wird so aus der Reaktionsmischung entfernt.

Es ist aber oft zweckmäßig, das Gemisch von Zucker und heterocyclischer Base nur mit der berechneten Menge an HMDS in Gegenwart von katalytischen Mengen an TCS zu silylieren, wobei nur Ammoniak entsteht und beim Erhitzen in Acetonitril entweicht. Bei dieser Reaktionsführung ist das Reaktionsgemisch nicht durch NH₄Cl verunreinigt und man kann die Lewissäure nach erfolgter Silylierung, wobei sich eine klare Lösung bildet, zusetzen.

Man kann aber auch an Stelle von HMDS-TCS beliebige andere Silylierungsmittel wie z. B. N,O-Bistrimethylsilylacetamid oder N,O-Bistrimethylsilyltrifluoracetamid verwenden.

Als freie Zucker eignen sich die für die Nucleosidsynthese üblicherweise verwendeten Zucker wie D-Ribose, D-Xylose, D-Glucose, D-Galaktose u.a..

Bei der Reaktion von freier D-Ribose wird selektiv zuerst die primäre 5-Hydroxylgruppe der Furanoseform silyliert, so daß bei der Persilylierung praktisch nur die persilylierte D-Ribofuranose entsteht, aus der sich mit silylierten heterocyclischen Basen nur die entsprechenden natürlichen D-Ribofuranosyl-nucleoside bilden.

In einzelnen Fällen kann es von Vorteil sein, die freien primären Hydroxylgruppen der Zucker wie z. B. die 5-Hydroxyl-Gruppe der D-Ribose vor der eigentlichen Nucleosidsynthese selektiv z. B. mit tert. Butyldiphenylsilylchlorid, tert. Butyldimethylsilylchlorid, Thexyldimethylsilylchlorid oder Tritylchlorid umzusetzen und diese partiell geschützten Zuckerderivate dann anschließend in Gegenwart der heterocyclischen Basen mit HMDS-TCS zu persylilieren und mit Lewissäuren in die entsprechenden persilylierten Nucleoside zu überführen.

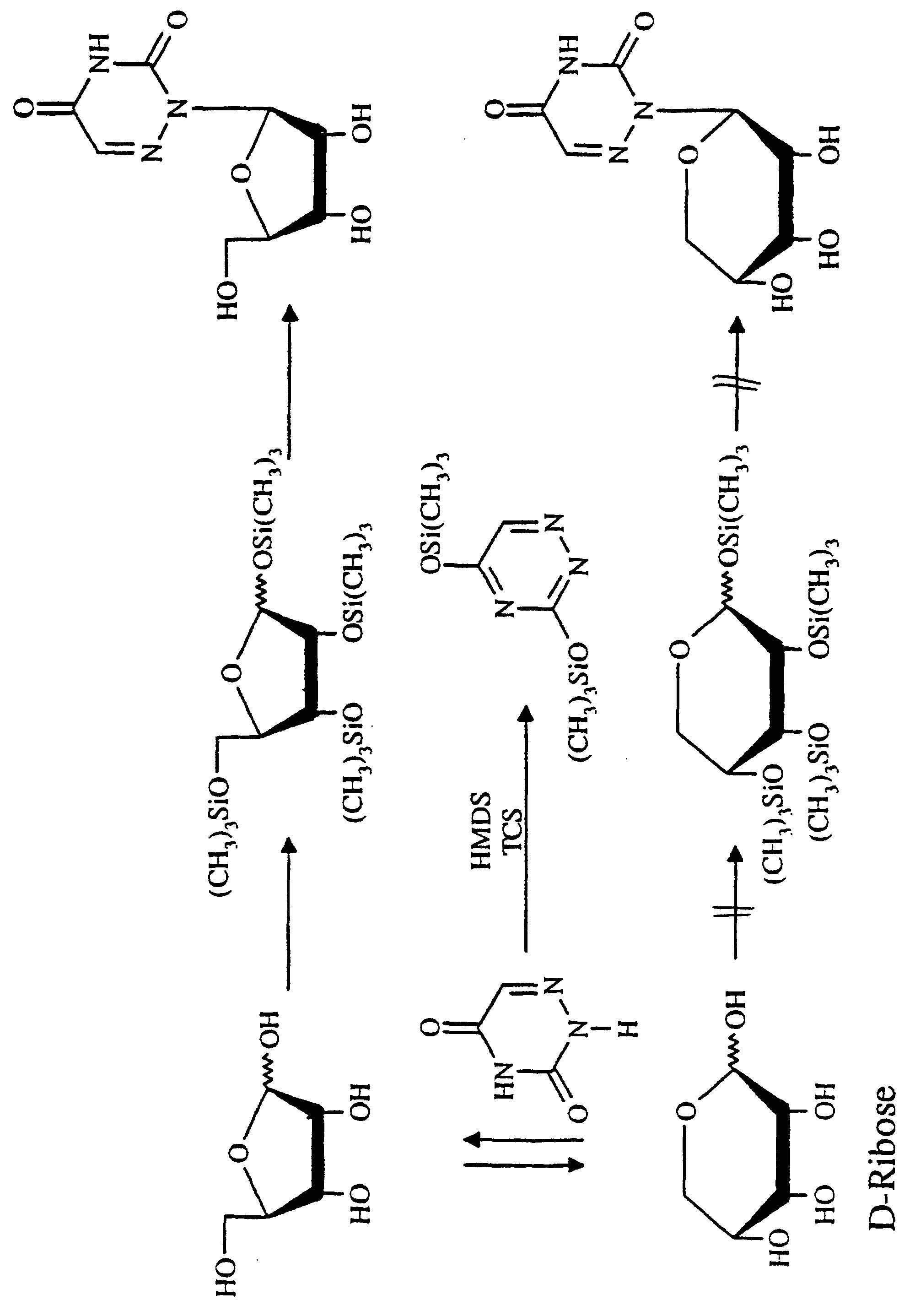

Als heterocyclische Basen werden Uracil, 6-Azauracil, Cytosin, N⁴-Acelylcytosin, Thymin, 2-Thiouracil, 6-Aza-2-thiouracil, N⁶-Benzoyladenin, N²-Acetyl- oder Isobutyryl-guanin sowie weitere Deaza- oder Azapurine bevorzugt.

Als unpolare absolute Lösungsmittel dienen vor allem Acetonitril, Nitromethan, 1,2-Dichloräthan, CS₂, Chloroform, Methylenchlorid, Tetrachloräthylen sowie Toluol, in denen bei der Persilylierung des Gemisches von freiem Zucker und freier heterocyclischer Base meist eine homogene Lösung entsteht.

Als Lewissäuren sind die vorne genannten Lewissäuren, aber auch alle anderen als Friedel-Crafts Katalysatoren bekannten Lewissäuren wie SnCl₄, SnCl₂, TiCl₄, BF₃-OEt₂, FeCl₃ usw.geeignet und führen zur Bildung von persilylierten Nucleosiden.

Bei der Aufarbeitung wird vor dem Abdampfen der Reaktionsmischung inAcetonitril (oder einem anderen indifferenten Lösungsmittel) noch überschüssiges Hexamethyldisilazan (HMDS) als Base zur Neutralisation der Lewissäure zugegeben. Der verbleibende Rückstand wird dann in überschüssigem Methanol in Gegenwart von Fluoridionen (KF oder tert. Butylammoniumfluorid-Hydrat) zur Transsilylierung ca. 1 - 3 h gekocht und abgedampft.

Die dabei entstehenden freien Uridine werden dann über eine Säule von stark basischen Austauschern wie Dowex 1 (OH-Form) filtriert. Bei der Elution mit verdünnten oder konzentriertem Ammoniak werden die bei der Nucleosidsynthese entstandenen neutralen Zucker eluiert, während die sauren Uridine erst mit 5 % Ameisensäure von der Säule ausgewaschen werden.

Die so gewonnenen rohen Uridine werden, falls sie noch nicht kristallisieren, z. B. mit Acetanhydrid-Pyridin acetyliert und nach Kodestillation mit Xylol im System Toluol-Essigester über eine Säule von Silicagel filtriert. Darauf kristallisieren die Acetate und lassen sich nach Umkristallisation aus Methanol oder Ethanol durch Behandlung mit methanolischen Ammoniak wieder in die reinen, freien Nucleoside zurückverwandeln.

Die analog synthetisierten Cytidine, Adenosine und Guanosine werden auf eine Säule von stark sauren Ionenaustauschen wie Dowex 50 oder IR-120 (H-Form) gegeben und nach Auswaschen der Oligosaccharide mit steigenden Konzentrationen von Ammoniak eluiert. Im Falle der Guanosine muß der Ionenaustauscher mit verd. HCl nachgewaschen werden.

Diese vorgereinigten Cytidine, Adenosine oder Guanosine werden, falls sie nicht kristallisieren, ebenfalls mit Acetanhydrid-Pyridin acetyliert, die Acetate über eine Säule von Silicagel gereinigt und die kristallisierten Acetate mit methanolischem Ammoniak in die freien, reinen Nucleoside zurückverwandelt.

In manchen Fällen ist es angebracht, auf die Ionenaustauschertrennung zu verzichten und das Rohprodukt nach der Transsilylierung direkt durch Acetylierung, Chromatographie an einer Säule von Silicagel, Umkristallisation und schließliche Verseifung mit methanolischem Ammoniak zu reinigen.

Weiterhin ist es vor allem bei der Synthese von Adenosinen möglich, die als Nebenprodukte entstandenen reduzierenden Oligosaccharide durch Behandlung mit Hydrazin oder Hydrazinhydrat bzw. Semicarbazid in polare Derivate zu überführen, die sich gut abtrennen lassen.

Die nachfolgenden Beispiele sollen das erfindungsgemäße Verfahren erläutern.

### Beispiel 1

5 mMol (0,56 g) Azauracil, 10 mMol (1,80 g) D-Glucose, 15 mMol (3,15 ml) HMDS, 15 mMol (1,89 ml) Trimethylchlorsilan und 25 ml Acetonitril werden zusammengegeben und am Rückfluß 5 Stunden gekocht. Dann wird noch 5 mMol = 1,05 ml HMDS, 5 mMol (0,63 ml) TCS dazugegeben und nochmal 5 Stunden gekocht. Der Kühler gewechselt und 7,5 mMol 1,12 g (0,66 ml) Trifluormethansulfonsäure dazugegeben und 3 Stunden am Rückfluß gekocht. 5 mMol HMDS werden dazugegeben, eingeengt, in CH₂Cl₂ aufgenommen und mit Eis/NaHCO₃ ausgeschüttelt, die org. Phase getrocknet, filtriert und eingeengt. 5,78 g in MeOH aufgenommen und am Rückfluß gekocht, dann eingeengt und der Rückstand (2,63 g) in H₂O über eine Austauschersäule von 50 ml Dowex 1 (OH-Form) 50 - 100 mesh gegeben. Elution mit 2500 ml 10% NH₃ und 3000 ml Wasser ergibt 0,85 g Oligosaccaride. Während Elution mit 750 ml 5% Ameisensäure 1,42 g rohes Nucleosid ergibt. Dieses wird mit 50 ml Pyridin und 25 ml Acetanhydrid 1 Stunde bei 80 °C erwärmt. Anschließend eingeengt 2 x mit Xylol abgedampft, in 100 ml CH₂Cl₂ gelöst mit 50 ml ges. NaHCO₃ -/Eis ausgeschüttelt → Emulsion über Celite filtriert mit CH₂Cl₂ + H₂O nachgewaschen. Phasen getrennt 3 x mit je 50 ml CH₂Cl₂ nachextrahiert. CH₂Cl₂-Phasen über Na₂SO₄ getrocknet, filtriert und eingeengt. Rohprodukt 1,65 g über 200 g Silicagel (230 - 400 mesh) mit Toluol/Essigester 1: 1 chromatographiert. Nach 1000 ml Vorlauf wurden in den folgenden 1000 ml 0,64 g ca. 30% 2-(2,3,4,6-Tetra-O-acetyl-ß-D-glucopyranosyl)-2,3,4,5-tetrahydro-1,2,4-triazine-3,5-dione eluiert. Umkristallisation aus Methanol gibt reines Tetraacetat mit einem Schmelzpuntk von 208 - 210 °C.

### Beispiel 2

5 mMol (0,56 g)Azauracil, 5 mMol (0,75 g) D-Ribose, 14 mMol (2,92 ml) HMDS, 14 mMol (1,77 ml) Trimethylchlorsilan und 50 ml Acetonitril werden zusammengegeben und 1 1/2 Stunden am Rückfluß gekocht. Anschließend auf + 10 ° abgekühlt und 7,5 mMol (0,66 ml) Trifluormethansulfonsäure zugegeben und 8 Stunden am Rückfluß gekocht. Nach Abkühlen auf 24 ° am Rotationsdampfer eingeengt und 30 Minuten mit 50 ml Methanol gekocht. Im Vakuum eingeengt, mit 20 ml Pyridin und 10 ml Essigsäureanhydrid 2 Stunden bei 60 ° gerührt.

Anschließend eingeengt und 2 x mit je 20 ml Xylol abdestilliert. Der Rückstand wird mit Toluol/Essigester 1 : 1an 100 g Silikagel (230 - 400 mesh) chromatographiert und ergibt 0,9 g rohes Azazauridintriacetat.

Umkristallisation aus Essigester/Hexan ergab 0,62 g ( = 33,4%) reines Azauridintriacetat mit Schmelzpunkt 102 - 103 °.

### Beispiel 3

Eine Mischung aus 15 mMol (2,25 g) trockener D-Ribose und 5 mMol (0,565 g) 6-Azuracil wird mit 15 ml HMDS und 0,1 ml Trimethylchlorsilan 2 Stunden in 30 ml kochendem absoluten Acetonitril silyliert. Nach Eindampfen im Vakuum wird der Rückstand 2 Stunden lang bei 80 °/1 mm gehalten, danach in 40 ml kochendem absoluten Acetonitril gelöst und 5,5 mMol (0,99 ml) Trimethylsilyltriflat in 10 ml Acetonitril innerhalb von 45 Minuten bei 80 °C hinzugefügt. Nach 7 Stunden bei 80 °C werden 20 ml einer gesättigten wässrigen NaHCO₃-Lösung bei 24 °C hinzugefügt und die resultierende dunkle Mischung für 4 Stunden bei 24 °C gerührt. Nach Einengung wird der dunkle Rückstand mit 3 x 100 ml kochendem Methanol extrahiert und die Extrakte eingeengt. Der Rückstand wird in ca. 10 ml Wasser gelöst und an ca. 20 g SiO₂ absorbiert, welches auf eine Säule von 200 g SiO₂ raufgegeben wird (vorbehandelt 4 Stunden lang mit 25% NH₃ (9 : 1), bis ein klares Filtrat erhalten wird). Die Elution mit Ethanol / 25% NH₃ (9:1) führt zu ersten 675 ml Eluat, das Zuckermengen wie auch Spuren von 6-Azauracil enthält. Die nachfolgenden 650 ml liefern 0,84 g (68,3%) leicht gefärbtes rohes 6-Azauridin, das in Wasser mit Aktivkohle behandelt wird und, aus Ethanol kristallisiert, reines kristallines 6-Azauridin (Schmelzpunkt 159 - 161 °C) liefert.

## Patentansprüche

1. Verfahren zur Herstellung von Nukleosiden, **dadurch gekennzeichnet, daß** freie Zucker mit heterocyclischen Basen in Gegenwart von Silylierungsmitteln in einem inerten Lösungsmittel mit einer Lewissäure umgesetzt werden.

2. Verfahren nach Anspruch 1, **dadurch gekennzeichnet, daß** als Silylierungsmittel N,O-Bistrimethylsilylacetamid oder N,O-Bistrimethylsilyltrifluoracetamid verwendet wird.

3. Verfahren nach Anspruch 1, **dadurch gekennzeichnet, daß** als Silylierungsmittel Hexamethyldisilazan und Trimethylchlorsilan verwendet werden.

4. Verfahren nach einem der Ansprüche 1 bis 3, **dadurch gekennzeichnet, daß** als Lewissäure Trimethylsilyltriflat, Trifluormethansulfonsäure, Trimethylsilylnonaflat, Perfluorbutansulfonsäure oder Friedel-Crafts Katalysatoren verwendet werden.

5. Verfahren nach Anspruch 4, **dadurch gekennzeichnet, daß** der Friedel-Crafts Katalysator ausgewählt wird aus SnCl₄, SnCl₂, TiCl₄, BF₃-OEt₂ und FeCl₃.

6. Verfahren nach einem der Ansprüche 1 bis 5, **dadurch gekennzeichnet, daß** das inerte Lösungsmittel ausgewählt wird aus Acetonitril, Nitromethan, 1,2-Dichlorethan, CS₂, Chloroform, Methylenchlorid, Tetrachloroethylen und Toluol.

7. Verfahren nach einem der Ansprüche 1 bis 6, **dadurch gekennzeichnet, daß** der Zucker ausgewählt wird aus D-Ribose, D-Xylose, D-Glukose und D-Galaktose.

8. Verfahren nach einem der Ansprüche 1 bis 7, **dadurch gekennzeichnet, daß** die heterocyclische Base ausgewählt wird aus Uracil, 6-Azauracil, Cytosin, N⁴-Acetylcytosin, Thymin, 2-Thiouracil, 6-Aza-2-Thiouracil, N⁶-Benzoyladenin, N²-Acetylguanin und Isobutyrylguanin.

## Claims

1. Process for preparing nucleosides, **characterized in that** free sugars with heterocyclic bases are reacted in the presence of silylating agents in an inert solvent containing a Lewis acid.

2. Process as claimed in Claim 1, **characterized in that** N,O-bis(trimethylsilyl)acetamide or N,O-bis(trimethylsilyl)trifluoroacetamide is used as the silylating agent.

3. Process as claimed in Claim 1, **characterized in that** hexamethyldisilazane and trimethylchlorosilane are used as the silylating agent.

4. Process as claimed in one of Claims 1 to 3, **characterized in that** trimethylsilyl triflate, trifluoromethanesulphonic acid, trimethylsilyl nonaflate, perfluorobutanesulphonic acid or Friedel-Crafts catalysts is/are used as the Lewis acid.

5. Process as claimed in Claim 4, **characterized in that** the Friedel-Crafts catalyst is selected from SnCl₄, SnCl₂, TiCl₄, BF₃-Oet₂ and FeCl₃.

6. Process as claimed in one of Claims 1 to 5, **characterized in that** the inert solvent is selected from acetonitrile, nitromethane, 1,2-dichloroethane, CS₂, chloroform, methylene chloride, tetrachloroethylene and toluene.

7. Process as claimed in one of Claims 1 to 6, **characterized in that** the sugar is selected from D-ribose, D-xylose, D-glucose and D-galactose.

8. Process as claimed in one of Claims 1 to 7, **characterized in that** the heterocyclic base is selected from uracil, 6-azauracil, cytosine, N⁴-acetylcytosine, thymine, 2-thiouracil, 6-aza-2-thiouracil, N⁶-benzoyladenine, N²-acetylguanine and isobutyrylguanine.

## Revendications

1. Procédé de préparation de nucléosides, **caractérisé en ce que** l'on fait réagir des sucres libres avec des bases hétérocycliques en présence d'agents de silylation dans un solvant inerte avec un acide de Lewis.

2. Procédé selon la revendication 1, **caractérisé en ce que** l'on utilise comme agent de silylation du N,O-bistriméthylsilylacétamide ou du N,O-bistriméthylsilyltrifluoroacétamide.

3. Procédé selon la revendication 1, **caractérisé en ce que** l'on utilise comme agent de silylation de l'hexaméthyldisilazane et du triméthylchlorosilane.

4. Procédé selon l'une des revendications 1 à 3, **caractérisé en ce que** l'on utilise comme acide de Lewis du triflate de triméthylsilyle, de l'acide trifluorométhanesulfonique, du nonaflate de triméthylsilyle, de l'acide perfluorobutanesulfonique ou des catalyseurs de Friedel-Crafts.

5. Procédé selon la revendication 4, **caractérisé en ce que** le catalyseur de Friedel-Crafts est choisi parmi SnCl₄, SnCl₂, TiCl₄, BF₃-OEt₂ et FeCl₃.

6. Procédé selon l'une des revendications 1 à 5, **caractérisé en ce que** le solvant inerte est choisi parmi l'acétonitrile, le nitrométhane, le 1,2-dichloroéthane, le CS₂, le chloroforme, le chlorure de méthylène, le tétrachloroéthylène et le toluène.

7. Procédé selon l'une des revendications 1 à 6, **caractérisé en ce que** le sucre est choisi parmi le D-ribose, le D-xylose, le D-glucose et le D-galactose,

8. Procédé selon l'une des revendications 1 à 7, **caractérisé en ce que** la base hétérocyclique est choisie parmi l'uracile, le 6-azauracile, la cytosine, la N⁴-acétylcytosine, la thymine, le 2-thiouracile, le 6-aza-2-thiouracile, la N⁶-benzoyladénine, la N²-acétylguanine et l'isobutyrylguanine.
